# FASCICULE DE BREVET EUROPEEN

(11) **EP 2 452 658 B1**
(45) Date de publication et mention de la délivrance du brevet: **03.07.2013**
(21) Numéro de dépôt: 11186919.4
(22) Date de dépôt: 27.10.2011
(51) Int. Cl.: A61F 13/08

(54) **Orthèse sur mesure de compression/contention, pour le renforcement de la pompe musculo-aponévrotique du mollet**
Maßgeschneiderte Kompressions-/Stützorthese zur Verstärkung der Muskelfaszienpumpe der Wade
Tailor-made compression/containment orthosis for reinforcing the muscular-aponeurotic pump of the calf

(30) Priorité: 10.11.2010 FR 1059288
(43) Date de publication de la demande: 16.05.2012
(73) Titulaire: Innothera Topic International, 94110 Arcueil (FR)
(72) Inventeur: Cros, François, 94200 IVRY/SEINE (FR); Rafstedt, Pauline, 54000 NANCY (FR)
(74) Mandataire: Dupuis-Latour, Dominique

(56) Documents cités:
- EP-A2- 2 050 848
- FR-A1- 2 824 471

## Description

L'invention concerne les orthèses de compression veineuse élastique (CVE), qui sont indiquées dans les diverses manifestations cliniques d'insuffisance veineuse des membres inférieurs.

Ces orthèses, anciennement connues sous la dénomination de "bas (ou chaussettes) de contention" ou "collants de contention", sont des dispositifs médicaux textiles produisant un effet thérapeutique par compression des membres inférieurs, par opposition aux "bas de maintien" (ou encore "bas de soutien" ou "bas anti-fatigue") et aux "bas mode", qui ne sont pas des dispositifs médicaux à visée thérapeutique.

Les orthèses de CVE sont conçues pour produire un effet thérapeutique par compression du membre inférieur sur une étendue plus ou moins grande, habituellement avec un profil dégressif vers le haut à partir de la cheville. Selon le type d'orthèse, la pression mesurée à la cheville peut varier de 10 à plus de 36 mmHg (soit 13 à 48 hPa, le mmHg étant toutefois d'usage courant comme unité de mesure de pression dans le domaine de la phlébologie et de la compression médicale). Pour la France, les bas sont répartis selon le référentiel ASQUAL en quatre classes textiles, à savoir la classe I (13 à 20 hPa ≈ 10 à 15 mmHg à la cheville), la classe II (20 à 27 hPa ≈ 15 à 20 mmHg), la classe III (27 à 48 hPa ≈ 20 à 36 mmHg) et la classe IV (> 48 hPa ≈ > 36 mmHg). Ces classes de compression peuvent être différentes pour d'autres pays.

Pour permettre une compression forte des membres inférieurs, ces orthèses sont réalisées à partir d'une maille tricotée de texture plus ou moins serrée avec incorporation d'un fil de trame élastique, généralement un élasthanne guipé.

Plus précisément, sous l'effet de la mise en place sur le membre, le textile tendu de l'orthèse exerce une compression résultant de la force de rappel des fibres élastiques qui composent le matériau, et l'application de ces forces de rappel élastique sur le périmètre du contour engendre en un point donné, selon la loi de Laplace, une pression locale inversement proportionnelle au rayon de courbure du contour en ce point.

Cette pression est la "pression textile" telle que définie et calculée au sens de la norme française NF G 30-102, partie B. On désignera dans la présente description par "pression" la moyenne des pressions normalisées localement exercées à une altitude donnée le long d'un contour de la jambe (contour circulaire ou elliptique, dans l'approximation d'une jambe-modèle).

La maille et les fils, ainsi que le dimensionnement des rangées de maille, sont choisis de manière à appliquer des pressions prédéterminées à différentes altitudes du membre inférieur, par exemple à la hauteur de la cheville, au départ du mollet, au niveau du mollet, au creux poplité, etc. jusqu'en haut de cuisse, altitudes notées conventionnellement *B*, C ... G. Ces différentes pressions sont définies pour chaque classe en référence à des gabarits métrologiques tels que la jambe-modèle de la norme française NF G 30-102 partie B, annexe B, correspondant à la jambe-modèle "type Hohenstein" selon le référentiel allemand RAL-GZ 387.

La caractéristique évoquée plus haut de dégressivité du profil de pression consiste à exercer une pression maximale à la cheville puis dégressive de la cheville au mollet ou à la cuisse. Elle repose sur le fait qu'en situation orthostatique la pression intraveineuse est dégressive de la cheville au mollet puis jusqu'à la cuisse. Dans le cas d'une insuffisance veineuse chronique, la compression élastique exercée par l'orthèse sur le membre va induire un effet anti-stase favorisant le retour veineux.

Plus précisément, sur le plan physiologique, le mollet est l'élément-clef de l'hémodynamique veineuse des membres inférieurs et de la pathologie veineuse chronique.

On a décrit notamment l'importance de l'effet de la "pompe musculaire" ou "pompe musculo-aponévrotique du mollet" (PMAM) en termes de flux sanguin veineux de retour, où les cycles physiologiques de contractions et de relâchements des muscles du mollet provoquent, par le jeu des ouvertures et fermetures des valvules veineuses, les vidanges et remplissages du réseau veineux du membre inférieur. Le rendement de la PMAM se réduit progressivement avec l'âge des sujets, et de ce fait aggrave naturellement les insuffisances veineuses chroniques.

L'insuffisance veineuse chronique est caractérisée par une défaillance de cet effet de pompe musculaire. Lorsque cette insuffisance est sévère, la cheville est également impliquée, du fait des reflux profonds ou par les perforantes de Cockett, qui jouent un rôle majeur dans la genèse des troubles trophiques et des ulcères.

Le point de départ de l'invention est la recherche d'un moyen permettant d'améliorer le rendement de la PMAM, voire de la suppléer, grâce à une orthèse compressive mieux adaptée à ce rôle que les orthèses qui ont pu être proposées jusqu'à présent.

En effet, le préjugé selon lequel le bas devrait exercer une pression maximale à la cheville, puis dégressive de la cheville au mollet ou à la cuisse, repose sur l'idée des répartitions des pressions intraveineuses en situation orthostatique. En effet, dans cette situation, par l'effet de la gravité la pression intraveineuse est dégressive de la cheville à la cuisse, d'où la nécessité d'une contrepression correspondante.

Or l'étude de la physiologie veineuse, notamment par les outils récents de modélisation et de simulation de contention tels que ceux décrits dans le WO 2006/087442 A1 (Laboratoires Innothera), montre que l'efficacité d'une orthèse de CVE réside plutôt, dès lors que l'on est capable de faire fonctionner sa PMAM, dans l'amélioration de son rendement.

Le FR 2 824 471 B1 (Rodier) décrit une approche consistant à réaliser une "compression/contention élective" au moyen d'un bas multizone à tricotage différencié, associant une région en maille très élastique au niveau du pied et de la cheville, suivie par une région en maille peu élastique depuis le bas du mollet jusqu'au creux poplité, et prolongée par une région en maille à nouveau très élastique depuis le genou jusqu'en haut de cuisse. L'idée de base consiste à prévoir des zones à effet plutôt compressif (pied, cheville et cuisse) de part et d'autre d'une zone à effet plutôt contentif (mollet). Cette dernière zone de l'orthèse produira moins d'effet au repos que celles qui l'entourent, en revanche lors des contractions du muscle du mollet elle exercera une compression accrue, augmentant la puissance et renforçant l'effet de vidange de la PMAM.

Il convient de préciser à cet égard que les termes de "compression" et de "contention" définissent des effets bien différents, bien qu'ils soient parfois confondus dans le langage commun :
- la "compression" est l'effet produit par une orthèse élastique, aussi bien au repos qu'à l'effort, sur un segment de membre du fait des forces plus ou moins puissantes de rappel des fibres élastiques de cette orthèse. Ces forces agissent de manière quasi-constante sur le membre : au repos, la compression est présente à la valeur de pression nominale, et à l'effort l'effet de cette compression est augmenté par la contraction des masses musculaires ;
- à l'inverse, la "contention" est l'effet produit par une orthèse qui agit de manière différenciée (effort/repos) sur un segment de membre sous l'action d'une structure considérée comme inélastique, par exemple un bandage non élastique, également désigné "bandage à allongement court". Au repos, ce type de bande exerce une pression faible, voire nulle ; en revanche, pendant la contraction musculaire, elle s'oppose aux augmentations locales de volume du mollet qui vient buter sur la structure non élastique, la pression se trouvant ainsi augmentée. La contention est donc efficace et active à l'effort et quasi-inactive au repos.

C'est pour désigner ces deux notions différentes que seront utilisés dans la suite les deux termes respectifs de "compression" (ou "compressif") et de "contention" (ou "contentif").

Au regard de ces définitions, la proposition du FR 2 824 471 B1 précité, qui ne met en oeuvre que des fils et des mailles plus ou moins élastiques sur la hauteur de l'orthèse, ne produit qu'un effet contentif très partiel au niveau du mollet.

Il s'agit plutôt de zones toutes élastiques mais à élasticité différenciée, comme cela a pu être proposé par ailleurs par les EP 0 934 043 B1 (Couzan) ou EP 1 240 880 A2 (Stolk). Ces deux derniers documents enseignent de réaliser un bas ou une chaussette avec une zone moins rigide (plus élastique) dans la région du mollet, respectivement de façon uniforme sur toute la circonférence du mollet, ou seulement dans la région postérieure de celui-ci.

En outre, du point de vue de la technologie, toutes ces structures "multizone" de l'art antérieur s'avèrent difficiles à réaliser en pratique, compte tenu de la difficulté qu'il y a à régler la machine de tricotage pour obtenir les profils d'élasticité variable requis, avec des transitions très abruptes entre des textures très hétérogènes correspondant aux différentes zones du bas ou de la chaussette.

D'autre part et surtout, ces orthèses que l'on pourrait qualifier de "semi-contentives" ne sont pas adaptées spécifiquement à un patient donné. Concrètement, le praticien se contente de sélectionner une orthèse dans une grille de tailles après avoir mesuré le périmètre de la cheville et du mollet. Ceci aboutit en pratique à une solution de compromis qui ne tient pas compte de la morphologie réelle du mollet, qui peut être très variable d'un patient à l'autre et qui ne peut être décrite convenablement par une unique mesure du périmètre maximal du mollet.

Cet inconvénient est particulièrement accru dans le cadre de produits censés produire un effet contentif, car le renforcement de l'effet de PMAM est subordonné à un ajustement précis de la structure non élastique au segment de membre concerné, sur toute l'étendue de celui-ci : si la structure non élastique n'est pas en contact étroit avec le membre au repos, elle ne procurera que très peu d'effet pour une augmentation faible ou modérée du volume du muscle ; si au contraire sa dimension est trop faible elle exercera une contrainte sur le membre même au repos, avec des effets délétères sur la circulation sanguine, outre une sensation de carcan risquant de rendre le port de l'orthèse particulièrement inconfortable pour le patient.

Il apparaît ainsi souhaitable de pouvoir réaliser des orthèses procurant un véritable effet contentif sur le mollet grâce à une structure non élastique (et non pas une structure à élasticité moindre), adaptée à la morphologie exacte du segment de membre de chaque patient.

Si l'on veut disposer d'un produit contentif rigide sur mesure, adapté spécifiquement au patient, un première solution consiste à utiliser des bandages multicouche, avec la difficulté bien connue qu'il y a à bien ajuster le bandage, ni trop serré (il entraînerait un écrasement du mollet) ni trop lâche (il ne produirait plus aucun effet), d'où un résultat très "opérateur-dépendant". Comme expliqué plus haut, l'ajustement d'un produit rigide de contention est très critique, à la différence d'une structure élastique compressive, beaucoup plus tolérante.

En outre, le bandage doit être refait régulièrement, à chaque fois avec le même soin pour un bon ajustement.

Pour ces raisons, les patients préfèrent généralement recourir à une autre solution, sous forme d'orthèse tricotée à enfiler, plus commode et plus esthétique.

Il s'agit alors de fabriquer un produit rigide sur mesure, parfaitement adapté à la morphologie particulière du patient. La technique consiste à prendre les mesures du mollet de la façon la plus complète, avec des cotes à plusieurs altitudes. L'orthèse est ensuite tricotée sur un métier à plat, puis mise en forme par réalisation d'une couture sur tout son long, ce qui nécessite une étape de confection supplémentaire. On comprendra que cette technique de sur-mesure intégral est longue à mettre en oeuvre, compliquée et donc coûteuse et ne permet pas une grande diffusion des produits rigides de contention, malgré leurs avantages thérapeutiques évidents.

Le problème de l'invention est ainsi de pouvoir réaliser une orthèse de contention (produit rigide) qui puisse se présenter sous forme d'un produit final "sur mesure", donc parfaitement ajusté à la morphologie du patient, mais qui pour autant ne nécessite pas une fabrication par des techniques conventionnelles de "sur mesure", longues et coûteuses.

On verra en particulier que l'invention peut être mise en oeuvre (i) par un métier circulaire (et non un métier à plat, qui nécessiterait une étape de confection supplémentaire pour la réalisation de la couture) (ii) réalisant un produit standard, donc avec possibilité de fabrication à un coût raisonnable et en grande série.

Et ceci avec une nouvelle structure d'orthèse de CVE:
- qui assure un renforcement des effets bénéfiques de la PMAM par une contention appropriée du mollet,
- qui soit technologiquement aisée à réaliser,
- et qui puisse être facilement adaptée à des morphologies de jambe très différentes rencontrées dans la population des patients concernés.

On verra également que l'invention permet d'obtenir une orthèse de CVE du membre inférieur qui applique au niveau du mollet non plus une compression plus ou moins renforcée, mais une véritable contention, en plaçant autour du mollet un élément essentiellement rigide, c'est-à-dire non élastiquement déformable. En outre, à cette rigidité forte au mollet (effet de contention) sera associée une rigidité faible à la cheville (effet de compression).

En effet, une rigidité forte au mollet est considérée comme un moyen d'optimisation de la PMAM, qui est le moteur principal du retour veineux dans les membres inférieurs.

La rigidité forte au mollet doit être associée à une rigidité faible (donc une déformabilité élevée) en cheville pour garantir une facilité de mise en place, de retrait et de tolérance du produit - notamment pour éviter une compression trop forte, qui serait rapidement intolérable pour notamment un patient alité ou inactif.

La "rigidité" est ici entendue au sens de la définition de la prénorme européenne XP ENV 12718:2001, c'est-à-dire l'*"augmentation de compression par centimètre d'augmentation de circonférence de la jambe, exprimée en hectopascals par centimètre et*/*ou en millimètres de mercure par centimètre ".*

L'idée de base de l'invention consiste à réaliser une orthèse compressive par des techniques conventionnelles, mais en intégrant au produit une partie contentive, obtenue par incorporation dans la partie de l'orthèse qui englobe le mollet d'une fibre "intelligente" dont les caractéristiques mécaniques pourront être modifiées par une action ultérieure, action thermique par exemple.

Dans son état originel (avant activation), la structure sera souple, ce qui permettra de tricoter l'orthèse de manière classique, au moyen d'un métier circulaire.

Après activation, par exemple par chauffage, la fibre "intelligente" est étirable, ce qui permet la mise en place aisée du produit et garantit en particulier une parfaite adaptation à la forme du mollet du patient. Elle se transforme en fibre rigide, par exemple après refroidissement. Le produit résultant est donc parfaitement adapté à la forme du mollet du patient, et permet donc d'appliquer au mollet une contention efficace, grâce à la partie contentive dont la forme sera ajustée à la morphologie du mollet, cette partie étant en quelque sorte "moulée en place" sur le mollet du patient. Cette partie contentive de la région du mollet sera associée à une partie compressive conventionnelle sur le reste de la jambe, tout particulièrement dans la région de la cheville.

Dans un domaine voisin mais différent, le EP 0 272 989 A1 (Richard Frères SA) propose de soumettre une zone d'un article textile élastique de maintien (par exemple une genouillère) à un apport de chaleur localisé de manière à produire une rétraction des fils de tricot synthétiques dans cette zone. Cet apport de chaleur est opéré seulement sur une face de l'article, par exemple par apposition d'une plaque chauffante, la face opposée restant intacte. Le retrait thermique du fil synthétique de tricot provoque alors un blocage du fil (élastique) de trame inséré entre les mailles du tricot et par voie de conséquence une réduction ou suppression de l'élasticité du textile sur la face exposée à la chaleur, tout en conservant cette élasticité sur la face opposée. Cette technique permet notamment de réaliser des ouvertures pour les parties saillantes des articulations, par exemple autour de la rotule pour une genouillère. On peut ainsi assurer autour de la rotule un maintien différent du reste de l'article, ce qui permet d'isoler la rotule du reste de la genouillère tout en ayant une ouverture qui plaque bien contre cette rotule.

Plus précisément, l'invention propose une orthèse de CVE de même finalité que le FR 2 824 471 B1 précité, c'est-à-dire une orthèse de compression médicale en forme de chaussette, de bas ou de collant destinée à agir spécifiquement sur la PMAM.

Une telle orthèse comporte, de manière en elle-même connue, (i) une partie distale compressive élastique, apte à couvrir la cheville en s'étendant jusqu'avant le début du mollet, au niveau du point de jonction entre le tendon d'Achille et les muscles du mollet, et (ii) une partie proximale contentive, prolongeant la partie distale compressive et attenante à celle-ci, de manière à envelopper sur sa périphérie une région du mollet comprise entre le niveau du point de jonction entre le tendon d'Achille et les muscles du mollet et le niveau situé en-dessous de la tubérosité tibiale.

La partie distale est réalisée par tricotage d'un fil de tricot et d'un fil de trame, le dimensionnement et la nature des fils de tricot et de trame ainsi que la structure de maille étant choisis de manière à exercer en direction circonférentielle, après que l'orthèse ait été mise en place sur le membre, une force de rappel élastique propre à produire une compression du membre à un niveau de pression thérapeutique désirée. La partie proximale contentive est quant à elle une partie tubulaire déformable tricotée en continuité avec la partie distale compressive élastique.

De façon caractéristique de l'invention, la partie proximale contentive est essentiellement non élastique, et elle est réalisée par tricotage d'un fil de trame et d'un fil de tricot, l'un desdits fils de trame ou de tricot comprenant un fil thermoformable.

Très avantageusement, la partie distale compressive est réalisée par tricotage d'un fil de trame et d'un premier fil de tricot, et la partie proximale attenante est réalisée par tricotage du même fil de trame et d'un second fil de tricot comprenant ledit fil thermoformable, ce second fil de tricot étant utilisé en remplacement ou en complément dudit premier fil de tricot.

Le fil de tricot peut notamment être un élasthanne guipé polyamide et/ou coton, et le fil de trame un élasthanne guipé polyamide et/ou coton.

La partie proximale peut être une partie présentant au niveau de la circonférence maximale du mollet une forte rigidité, de 15 ± 2 mmHg/cm (≈ 20 ± 2 hPa/cm), ou une rigidité modérée, de 5 ± 2 mmHg/cm (≈ 7 ± 2 hPa/cm).

La partie distale compressive élastique peut être une partie faiblement compressive apte à exercer une pression de 10 à 20 mmHg (13 à 27 hPa), ou bien modérément compressive apte à exercer une pression de 20 à 30 mmHg (27 à 40 hPa), au niveau de la circonférence minimale de la cheville.

L'invention vise également un procédé spécifique de mise à la mesure de la jambe d'un patient d'une orthèse de compression/contention médicale du membre inférieur.

Ce procédé comprend les étapes suivantes : obtention d'une orthèse telle que ci-dessus ; chauffage de l'orthèse à une température au moins égale à la température d'activation du matériau du fil thermoformable pendant une durée prédéterminée, l'orthèse étant alors dans un état librement allongeable ; enfilage de l'orthèse sur la jambe du patient ; refroidissement de l'orthèse en place sur la jambe du patient ; et retrait de l'orthèse dans son état fini. L'orthèse dans son état fini présente une partie proximale contentive rendue rigide par ladite activation, et dont la forme et les dimensions se trouvent ajustées aux dimensions correspondantes du mollet du patient, permettant à cette partie proximale contentive de venir épouser parfaitement la forme du mollet.

Si le chauffage de l'orthèse précède l'enfilage sur la jambe, l'étape de chauffage est mise en oeuvre au moyen d'une forme chauffante sur laquelle l'orthèse a été préalablement mise en place, l'orthèse une fois chauffée étant retirée de cette forme pour être enfilée sur la jambe.

Inversement, si l'enfilage sur la jambe précède le chauffage de l'orthèse, l'étape de chauffage est mise en oeuvre au moyen d'une source de chaleur dirigée vers la jambe sur laquelle l'orthèse a été préalablement enfilée.

L'invention vise également un procédé spécifique de fabrication d'une orthèse de compression/contention médicale du membre inférieur telle que ci-dessus. Ce procédé comprend des étapes successives, exécutées en continuité, de tricotage de la partie distale compressive élastique à partir d'un fil de tricot et d'un fil de trame, et de tricotage de la partie proximale contentive à partir d'un fil de trame et d'un fil de tricot, l'un desdits fils de trame ou de tricot de cette partie proximale contentive comprenant un fil thermoformable.

Avantageusement, le procédé comprend : le tricotage de la partie distale compressive élastique à partir d'un fil de trame et d'un premier fil de tricot ; et le tricotage de la partie proximale contentive à partir du même fil de trame et d'un second fil de tricot comprenant ledit fil thermoformable, ce second fil de tricot étant utilisé en remplacement ou en complément dudit premier fil de tricot.

On va maintenant décrire un exemple de mise en oeuvre de l'invention, en référence aux dessins annexés où les mêmes références numériques désignent d'une figure à l'autre des éléments identiques ou fonctionnellement semblables.
La Figure 1 est une vue d'ensemble d'une orthèse selon l'invention, à l'état libre.
La Figure 2 est une vue en élévation de cette même orthèse enfilée sur un membre, avec indication des diverses altitudes normalisées auxquelles sont mesurées les pressions appliquées par l'orthèse sur le membre.
La Figure 3 est une vue macroscopique décrivant la structure de la zone de transition entre les parties proximale contentive non élastique et distale compressive élastique de l'orthèse.
La Figure 4 illustre les étapes successives du procédé de mise en oeuvre selon l'invention, destiné à mettre l'orthèse de l'invention à la mesure de la jambe du patient.
La Figure 5 est une variante de mise en oeuvre du procédé de la Figure 4.

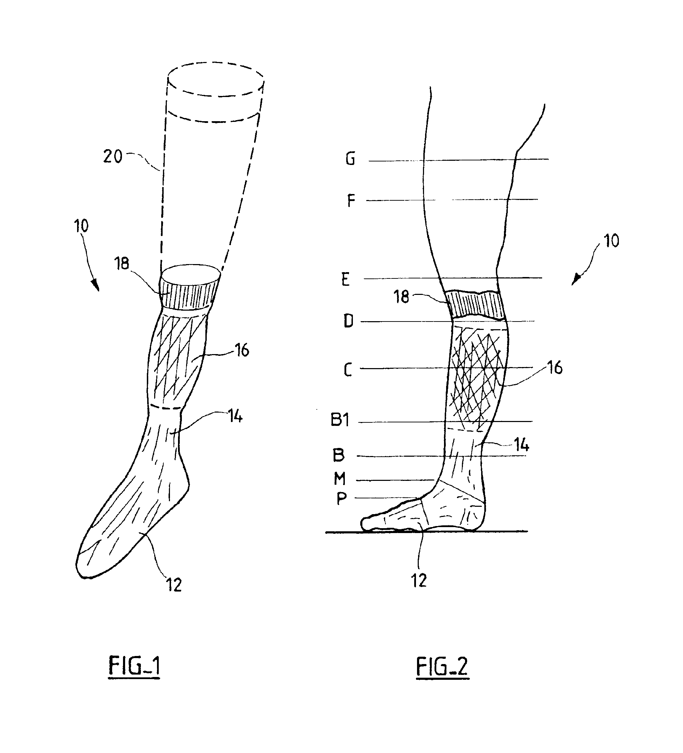

Sur les Figures 1 et 2, la référence 10 désigne de façon générale l'orthèse de l'invention, qui est une orthèse tricotée réalisée selon des procédés conventionnels sur un métier circulaire. Cette orthèse 10 de forme tubulaire comprend une partie 12 enveloppant le pied et une partie de jambe avec une partie distale 14 enveloppant la cheville et une partie proximale 16 enveloppant le mollet. L'ensemble s'étend jusqu'à un niveau situé au-dessous du genou, dans le cas où l'orthèse est une chaussette "mi-bas" (ou "bas-jarret"). Dans ce dernier cas, l'orthèse est terminée par une partie tricotée terminale de type "bord-côtes" 18.

Cette configuration en forme de chaussette n'est pas limitative, et l'invention peut être également réalisée sous forme d'un "bas-cuisse", prolongé par une partie de cuisse compressive 20. L'orthèse de l'invention peut être également réalisée sous forme d'un collant, et/ou dépourvue de partie de pied 12 (bas ou collant de type "pied ouvert").

Les différentes parties attenantes de l'orthèse que l'on vient de décrire sont tricotées en continu sur le métier circulaire, c'est-à-dire que la réalisation de cette orthèse ne requiert aucune étape de confection pour l'assemblage de pièces distinctes, à l'exception bien entendu des opérations de couture de la pointe au niveau de la partie de pied 12, si cette dernière est présente.

Sur la Figure 2, on a représenté les diverses altitudes du membre inférieur telles que définies par le référentiel morphologique indiqué en introduction (jambe-modèle "type Hohenstein") utilisant les notations normalisées :
B: cheville, au point de sa circonférence minimale ;
B1: point de jonction entre le tendon d'Achille et les muscles du mollet ;
C: mollet, au point de sa circonférence maximale;
D: juste au-dessous de la tubérosité tibiale (c'est-à-dire juste au-dessous du genou) ;
E: au centre de la rotule et au-dessus de l'arrière du genou (c'est-à-dire au niveau du creux poplité);
F: en milieu de cuisse ; et
G: en haut de cuisse.

Le mollet est le segment de membre compris entre les niveaux B1 et D, et la cheville est le segment de membre situé au-dessous du niveau B1.

La pression exercée à l'altitude B (au périmètre minimal de la cheville) est la pression prescrite pour la classe normalisée choisie (I, II, III ou IV). Les valeurs de pression peuvent être relevées par exemple sur dynamomètre conformément à la norme précitée NF G 30-102 partie B, après avoir enfilé l'orthèse sur un gabarit de référence tel que le modèle de jambe Hohenstein prescrit par cette norme.

La pression exercée au niveau de la cheville au point de sa circonférence minimale (niveau B) par la partie distale compressive élastique 14 doit être une pression thérapeutique efficace. Les valeurs suivantes peuvent être retenues, en fonction des besoins du patient :
- 10 à 20 mmHg (13 à 27 hPa) pour une compression relativement faible de la cheville ;
- 20 à 30 mmHg (27 à 40 hPa) pour une compression modérée de la cheville.

La partie distale compressive élastique 14 produisant ces pressions thérapeutiques est réalisée à partir d'une maille tricotée de texture plus ou moins serrée avec incorporation d'un fil de trame élastique, par exemple en utilisant :
- comme fil de trame, un fil tel qu'un élasthanne ou un mélange d'élasthanne et d'élasto-diène (latex de caoutchouc synthétique), guipé polyamide et/ou coton ; et
- comme fil de tricot (fil de maille), également un élasthanne guipé polyamide et/ou coton, de préférence avec un titre (masse linéique) moindre que celui du fil de trame.

De façon caractéristique de l'invention, la partie proximale 16 est une partie contentive (c'est-à-dire essentiellement non élastique après activation), de forme tubulaire, s'étendant :
- en direction verticale : sur l'étendue du mollet, c'est-à-dire sur la région comprise entre le niveau B1 (jonction entre le tendon d'Achille et les muscles du mollet) et le niveau D (au-dessous du genou), ou tout au moins sur la majeure partie de cette région ; on notera que la cheville (région s'étendant autour du niveau B) ne fait jamais partie de cette région recouverte par la partie proximale 16 ; et
- en direction circonférentielle : sur toute la périphérie du mollet.

Cette partie non élastique est réalisée sur mesure, de la manière que l'on va exposer ci-dessous, c'est-à-dire qu'elle présente une configuration extérieure précisément ajustée à la forme et aux dimensions du mollet du patient. De la sorte, une fois l'orthèse enfilée sur le membre cette partie exercera l'effet de contention recherché, c'est-à-dire qu'au repos elle n'exercera essentiellement aucun effort de contention, mais qu'à l'effort elle opposera au membre une rigidité procurant l'effet contentif au niveau d'efficacité souhaité.

En ce qui concerne la rigidité R_{c} de cette partie proximale contentive 16, on peut retenir les valeurs suivantes (selon la prénorme européenne XP ENV 12718:2001 précitée) :
- pour une contention forte : R_{c} = 15 ± 2 mmHg/cm (≈ 20 ± 2 hPa/cm);
- pour une contention modérée : R_{c} = 5 mmHg/cm (≈ 7 hPa/cm).

Ces valeurs R_{c} sont mesurées à l'altitude C, c'est-à-dire au point de la circonférence maximale du mollet.

En jouant séparément, d'une part sur l'élasticité de la partie distale compressive 14 au niveau de la cheville, et d'autre part sur la rigidité de la partie proximale contentive 16 au niveau du mollet, il est possible de combiner plusieurs effets de compression/contention, par exemple:
- faible compression en cheville/forte contention au mollet ;
- compression modérée en cheville/forte contention au mollet;
- faible compression en cheville/contention modérée au mollet ; ou
- compression modérée en cheville/contention modérée au mollet.

Selon un autre aspect caractéristique de l'invention, la partie proximale contentive non élastique 16 est réalisée en incorporant sélectivement dans la région correspondant à cette partie un fil en polymère thermoformable, c'est-à-dire un fil qui peut s'étendre par apport de chaleur pendant une durée prédéterminée.

Très avantageusement, les deux parties distale 14 et proximale 16 sont tricotées en continu au cours d'une même séquence sur la machine de tricotage, ce qui évite toute étape de confection pour l'assemblage de parties rapportées.

On prévoira seulement, au passage de la frontière entre les deux parties 14 et 16, d'incorporer au tricotage un remplacement du fil de tricot par le fil thermoformable précité. Cette opération pourra être effectuée en continu, sans interrompre le fonctionnement du métier à tricoter.

Le fil de trame reste quant à lui le même pour les deux parties distale 14 et proximale 16.

Avec l'exemple de fils indiqué plus haut pour la partie distale compressive élastique 14, la partie proximale 16 sera ainsi tricotée avec :
- comme fil de trame, le fil d'élasthanne guipé polyamide et/ou coton, et
- comme fil de tricot, le fil d'élasthanne guipé polyamide et/ou coton de moindre titre, plus un deuxième fil en polymère thermoformable.

Un fil thermoformable convenant à la mise en oeuvre de l'invention est par exemple réalisé à base de *Thermoform LXN* (marque déposée) produit par la Société Orfit Industries. Il s'agit d'un fil constitué d'un polymère filé en un monofilament flexible de poly-ε-caprolactone, dont les propriétés mécaniques sont modifiables par application d'une température de 65° pendant environ une minute.

Ce fil présente l'avantage de pouvoir être utilisé comme un fil standard sur un métier à tricoter conventionnel, ce qui permet d'utiliser la machine de façon classique, sans modification particulière.

L'étape d'activation a pour effet de ramollir le monofilament qui devient souple tant qu'il est chauffé et se rigidifie après refroidissement.

Pendant l'activation, un textile incorporant ce fil thermoformable peut être moulé en place sur toute forme appropriée. Après refroidissement, le textile durcira et gardera la configuration de la forme sur laquelle il aura été appliqué. Le poly-ε-caprolactone présente l'avantage d'avoir une température d'activation relativement faible (60 à 70°C) et de rester moulable tant qu'il reste à une température située au-dessus de sa température de cristallisation, en particulier jusqu'à 40 à 45°C, ce qui donne une très grande latitude pour le moulage notamment sur une partie du corps sans risque de température excessive.

Le EP 2 050 848 A2 (Orfit Industries) décrit un textile hybride réalisé en imbriquant un tel fil thermoformable avec un fil non thermofusible constituant le support du textile. La fibre thermoformable assure l'aptitude au moulage, tandis que la fibre non thermoformable procure structure et support au textile et contribue à l'augmentation de la rigidité et de la stabilité du produit final obtenu.

Ce brevet décrit notamment la manière dont la rétraction initiale de la fibre thermoformable au moment de l'activation est compensée par la rigidité procurée par les fibres structurelles non thermoformables, ce qui permet de libérer le textile de toute contrainte interne indésirable après refroidissement, permettant à celui-ci de garder la configuration de la forme sur laquelle il a été appliqué.

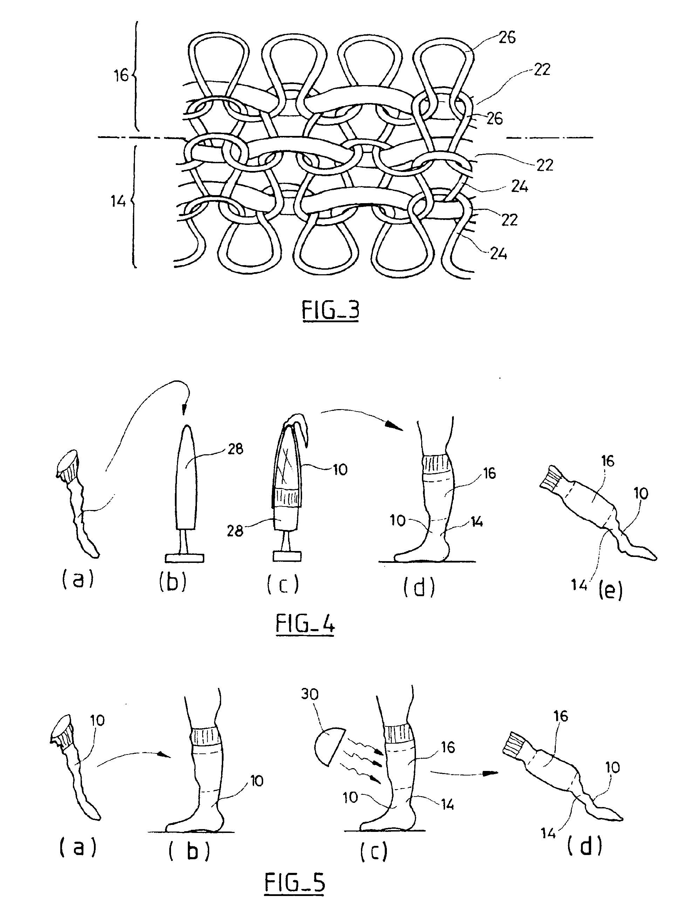

La Figure 3 illustre de manière plus précise la façon dont le fil thermoformable est incorporé à la structure de tricot pour réaliser la partie proximale contentive.

Cette figure montre la structure de maille à la frontière entre la partie distale compressive élastique 14 et la partie proximale contentive rigide 16. La transition se fait simplement par changement du fil de tricot, sans modification des paramètres de réglage de la machine de tricotage. la structure de maille est donc la même sur toute l'étendue de la partie de jambe de l'orthèse.

Le fil de trame 22 est le même sur les deux parties, par exemple un élasthanne double guipé polyamide. Le fil de tricot 24 de la partie élastique 14 est par exemple un élasthanne simple ou double guipé polyamide, tandis que le fil de tricot 26 de la partie rigide 16 est par exemple un fil assemblé composé d'un fil d'âme *Thermoform LXN* double guipé polyamide.

Le produit peut être tricoté suivant des techniques usuelles sur un métier circulaire classique, tel qu'un métier *Santoni.*

Les indications ci-dessus sont bien entendu données à titre d'exemple, sans aucun caractère limitatif.

La Figure 4 illustre le procédé de "mise en oeuvre au porter" permettant, à partir de l'orthèse de l'invention réalisée comme exposé ci-dessus, la mise à mesure de la partie proximale contentive 16, pour ajuster parfaitement celle-ci aux dimensions et au galbe du mollet du patient.

L'orthèse 10 qui vient d'être tricotée de la manière conventionnelle se présente initialement sous la forme d'un produit standard, c'est-à-dire un produit qui n'est pas sur mesure (étape a) ; il est seulement prévu, comme pour les orthèses de CVE classiques et même pour tout article d'habillement, des tailles standard appropriées, à choisir dans une grille dimensionnelle.

L'orthèse 10 est alors enfilée sur une lame chauffante 28 de forme allongée (étape b), qui va chauffer à une température de 60°C l'ensemble du produit, et notamment la partie contentive 16 (étape c). À cette température, la partie proximale contentive va prendre une consistance ramollie du fait de l'activation du fil thermoformable.

Le patient va alors enfiler l'orthèse (étape d) et la masser sur sa jambe et notamment sur son mollet, afin que la partie proximale 16 épouse parfaitement le galbe de celui-ci de la façon la plus uniforme possible.

Après refroidissement, l'orthèse peut être retirée (étape e). Elle aura pris sa forme définitive, "sur mesure", avec une partie proximale contentive 16 devenue rigide et ayant pris une forme épousant parfaitement le galbe et les dimensions du mollet du patient, et une partie distale compressive élastique 14, de sorte que l'on se trouve alors en présence d'un produit associant une rigidité forte au mollet (partie proximale contentive 16) et une rigidité faible à la cheville (partie distale compressive élastique 14) assurant une compression du membre inférieur à un niveau thérapeutique.

La Figure 5 illustre une variante de mise en oeuvre du procédé de mise à la mesure que l'on vient de décrire.

Dans ce cas, l'orthèse 10 dans son état initial (étape a) est enfilée telle quelle sur la jambe du patient (étape b), et c'est dans cette configuration que la chaleur est apportée (étape c), par un dispositif chauffant 30, par exemple un sèche-cheveux ou une source de rayonnement infrarouge, dirigé vers la partie proximale 16 enveloppant le mollet. Le patient masse l'orthèse sur sa jambe et la et laisse refroidir en place. Après refroidissement, l'orthèse peut être retirée et se retrouve alors dans son état final (étape d), mise à la mesure du patient.

## Revendications

1. Une orthèse de compression/contention médicale du membre inférieur en forme de chaussette, de bas ou de collant,
cette orthèse (10) comportant :
- une partie distale compressive (14) élastique, apte à couvrir la cheville en s'étendant jusqu'avant le début du mollet, au niveau du point de jonction entre le tendon d'Achille et les muscles du mollet,
cette partie distale étant réalisée par tricotage d'un fil de tricot et d'un fil de trame, le dimensionnement et la nature des fils de tricot et de trame ainsi que la structure de maille étant choisis de manière à exercer en direction circonférentielle, après que l'orthèse ait été mise en place sur le membre, une force de rappel élastique propre à produire une compression du membre à un niveau de pression thérapeutique désirée ; et
- une partie proximale contentive (16), prolongeant la partie distale compressive et attenante à celle-ci, de manière à envelopper sur sa périphérie une région du mollet comprise entre le niveau (B1) du point de jonction entre le tendon d'Achille et les muscles du mollet et le niveau (D) situé en-dessous de la tubérosité tibiale ;
cette partie proximale contentive étant une partie tubulaire déformable tricotée en continuité avec la partie distale compressive élastique, cette orthèse étant **caractérisée en ce que** la partie proximale contentive :
- est essentiellement non élastique, et
- est réalisée par tricotage d'un fil de trame et d'un fil de tricot, l'un desdits fils de trame ou de tricot comprenant un fil thermoformable.

2. L'orthèse de la revendication 1, dans laquelle la partie distale compressive est réalisée par tricotage d'un fil de trame et d'un premier fil de tricot, et la partie proximale attenante est réalisée par tricotage du même fil de trame et d'un autre fil de tricot comprenant ledit fil thermoformable.

3. L'orthèse de la revendication 2, dans laquelle ledit premier fil de tricot est un élasthanne guipé polyamide et/ou coton.

4. L'orthèse de la revendication 3, dans laquelle ledit fil de trame est un élasthanne guipé polyamide et/ou coton.

5. L'orthèse de la revendication 1, dans laquelle la partie proximale contentive est une partie présentant au niveau de la circonférence maximale du mollet une forte rigidité, de 15 ± 2 mmHg/cm (=20 ± 2 hPa/cm).

6. L'orthèse de la revendication 1, dans laquelle la partie proximale contentive est une partie présentant au niveau de la circonférence maximale du mollet une rigidité modérée, de 5 ± 2 mmHg/cm (= 7 ± 2 hPa/cm).

7. L'orthèse de la revendication 1, dans laquelle la partie distale compressive élastique est une partie faiblement compressive, apte à exercer une pression de 10 à 20 mmHg (13 à 27 hPa) au niveau de la circonférence minimale de la cheville.

8. L'orthèse de la revendication 1, dans laquelle la partie distale compressive élastique est une partie modérément compressive, apte à exercer une pression de 20 à 30 mmHg (27 à 40 hPa) au niveau de la circonférence minimale de la cheville.

9. Un procédé de mise à la mesure de la jambe d'un patient d'une orthèse de compression/contention médicale du membre inférieur, ce procédé comprenant les étapes suivantes :
- obtention d'une orthèse (10) selon l'une des revendications 1 à 8 dans un état initial brut de fabrication ;
- chauffage de l'orthèse à une température au moins égale à la température d'activation du matériau du fil thermoformable pendant une durée prédéterminée, l'orthèse étant alors dans un état librement allongeable ;
- enfilage de l'orthèse sur la jambe du patient ;
- refroidissement de l'orthèse en place sur la jambe du patient ; et
- retrait de l'orthèse dans son état fini,
l'orthèse dans son état fini présentant une partie proximale contentive (16) rendue rigide par ladite activation, et dont la forme et les dimensions se trouvent ajustées aux dimensions correspondantes du mollet du patient, permettant à cette partie proximale contentive de venir épouser parfaitement la forme du mollet.

10. Le procédé de mise à la mesure de la revendication 9, dans lequel l'étape de chauffage de l'orthèse précède l'étape d'enfilage sur la jambe, et dans lequel l'étape de chauffage est mise en oeuvre au moyen d'une forme chauffante (28) sur laquelle l'orthèse a été préalablement mise en place, l'orthèse une fois chauffée étant retirée de cette forme pour être enfilée sur la jambe.

11. Le procédé de mise à la mesure de la revendication 9, dans lequel l'étape d'enfilage sur la jambe précède l'étape de chauffage de l'orthèse, et dans lequel l'étape de chauffage est mise en oeuvre au moyen d'une source de chaleur (30) dirigée vers la jambe sur laquelle l'orthèse a été préalablement enfilée.

12. Un procédé de fabrication d'une orthèse de compression/contention médicale du membre inférieur selon l'une des revendications 1 à 8, comprenant les étapes successives suivantes, exécutées en continuité :
- tricotage de la partie distale compressive élastique à partir d'un fil de trame et d'un fil de tricot ; et
- tricotage de la partie proximale contentive à partir d'un fil de trame et d'un fil de tricot, l'un desdits fils de trame ou de tricot de cette partie proximale contentive comprenant un fil thermoformable.

13. Le procédé de la revendication 12, comprenant les étapes de :
- tricotage de la partie distale compressive élastique à partir d'un fil de trame et d'un premier fil de tricot ; et
- tricotage de la partie proximale contentive à partir du même fil de trame et d'un second fil de tricot comprenant ledit fil thermoformable, ce second fil de tricot étant utilisé en remplacement ou en complément dudit premier fil de tricot.

## Patentansprüche

1. Medizinische Kompressions-/Stützorthese der unteren Extremität in Form eines Kniestrumpfes, eines Strumpfes oder einer Strumpfhose,
wobei diese Orthese (10) Folgendes aufweist:
- einen elastischen distalen Kompressionsteil (14), der geeignet ist, den Knöchel zu bedecken, wobei er sich bis vor den Beginn der Wade auf der Höhe der Verbindungsstelle zwischen der Achillessehne und den Muskeln der Wade erstreckt,
wobei dieser distale Teil durch Stricken eines Strickfadens und eines Schussfadens hergestellt ist, wobei die Dimensionierung und die Beschaffenheit der Strick- und Schussfäden sowie die Maschenstruktur derart gewählt sind, dass in Umfangsrichtung, nachdem die Orthese auf der Extremität angebracht worden ist, eine elastische Rückholkraft ausgeübt wird, die geeignet ist, eine Kompression der Extremität mit einem Niveau entsprechend dem gewünschten therapeutischen Druck zu erzeugen; und
- einen proximalen Stützteil (16), der den distalen Kompressionsteil verlängert und an diesen angrenzt, so dass er auf seinem Umfang einen Bereich der Wade umhüllt, der zwischen der Höhe (B1) der Verbindungsstelle zwischen der Achillessehne und den Muskeln der Wade und der Höhe (D), die sich unterhalb des Schienbeinhöckers befindet, liegt;
wobei dieser proximale Stützteil ein verformbarer rohrförmiger Teil ist, der in Kontinuität mit dem elastischen distalen Kompressionsteil gestrickt ist,
wobei diese Orthese **dadurch gekennzeichnet ist, dass** der proximale Stützteil:
- im Wesentlichen nicht elastisch ist, und
- durch Stricken eines Schussfadens und eines Strickfadens hergestellt ist, wobei der Schuss- oder der Strickfaden einen thermoverformbaren Faden aufweist.

2. Orthese nach Anspruch 1, wobei der distale Kompressionsteil durch Stricken eines Schussfadens und eines ersten Strickfadens hergestellt ist und der angrenzende proximale Teil durch Stricken desselben Schussfadens und eines anderen Strickfadens, der den thermoverformbaren Faden aufweist, hergestellt ist.

3. Orthese nach Anspruch 2, wobei der erste Strickfaden ein mit Polyamid und/oder Baumwolle umsponnener Elastanfaden ist.

4. Orthese nach Anspruch 3, wobei der Schussfaden ein mit Polyamid und/oder Baumwolle umsponnener Elastanfaden ist.

5. Orthese nach Anspruch 1, wobei der proximale Stützteil ein Teil ist, der auf der Höhe des maximalen Umfangs der Wade eine hohe Steifigkeit aufweist, von 15 ± 2 mmHg/cm (≈ 20 ± 2 hPa/cm).

6. Orthese nach Anspruch 1, wobei der proximale Stützteil ein Teil ist, der auf der Höhe des maximalen Umfangs der Wade eine mäßige Steifigkeit aufweist, von 5 ± 2 mmHg/cm (≈ 7 ± 2 hPa/cm).

7. Orthese nach Anspruch 1, wobei der elastische distale Kompressionsteil ein schwach kompressiver Teil ist, der geeignet ist, einen Druck von 10 bis 20 mmHg (13 bis 27 hPa) auf der Höhe des minimalen Umfangs des Knöchels auszuüben.

8. Orthese nach Anspruch 1, wobei der elastische distale Kompressionsteil ein mäßig kompressiver Teil ist, der geeignet ist, einen Druck von 20 bis 30 mmHg (27 bis 40 hPa) auf der Höhe des minimalen Umfangs des Knöchels auszuüben.

9. Verfahren zur maßgeschneiderten Anpassung einer medizinischen Kompressions-/Stützorthese der unteren Extremität an das Bein eines Patienten, wobei dieses Verfahren die folgenden Schritte aufweist:
- Beschaffen einer Orthese (10) nach einem der Ansprüche 1 bis 8 in einem dem Herstellungszustand entsprechenden Anfangszustand;
- Erwärmen der Orthese auf eine Temperatur, die mindestens gleich der Aktivierungstemperatur des Materials des thermoverformbaren Fadens während einer vorgegebenen Zeitdauer ist, wobei sich die Orthese dann in einem frei dehnbaren Zustand befindet;
- Aufziehen der Orthese auf das Bein des Patienten;
- Abkühlen der Orthese an Ort und Stelle auf dem Bein des Patienten; und
- Abziehen der Orthese in ihrem Endzustand,
wobei die Orthese in ihrem Endzustand einen proximalen Stützteil (16) aufweist, der durch die Aktivierung steif geworden ist und dessen Form und Abmessungen sich als an die entsprechenden Abmessungen der Wade des Patienten angepasst erweisen, was es diesem proximalen Stützteil ermöglicht, sich perfekt an die Form der Wade anzuschmiegen.

10. Verfahren zur maßgeschneiderten Anpassung nach Anspruch 9, wobei der Schritt des Erwärmens der Orthese dem Schritt des Aufziehens auf das Bein vorangeht, und wobei der Schritt des Erwärmens mittels einer Heizform (28) durchgeführt wird, auf welcher die Orthese zuvor angebracht worden ist, wobei die Orthese, nachdem sie erwärmt worden ist, von dieser Form abgezogen wird, um auf das Bein aufgezogen zu werden.

11. Verfahren zur maßgeschneiderten Anpassung nach Anspruch 9, wobei der Schritt des Aufziehens auf das Bein dem Schritt des Erwärmens der Orthese vorangeht, und wobei der Schritt des Erwärmens mittels einer Wärmequelle (30) durchgeführt wird, die auf das Bein gerichtet wird, auf welches die Orthese zuvor aufgezogen worden ist.

12. Verfahren zur Herstellung einer medizinischen Kompressions-/Stützorthese der unteren Extremität nach einem der Ansprüche 1 bis 8, welches die folgenden aufeinanderfolgenden Schritte aufweist, die in Kontinuität ausgeführt werden:
- Stricken des elastischen distalen Kompressionsteils aus einem Schussfaden und einem Strickfaden; und
- Stricken des proximalen Stützteils aus einem Schussfaden und einem Strickfaden, wobei einer von dem Schuss- oder Strickfaden dieses proximalen Stützteils einen thermoverformbaren Faden aufweist.

13. Verfahren nach Anspruch 12, welches die folgenden Schritte aufweist:
- Stricken des elastischen distalen Kompressionsteils aus einem Schussfaden und einem Strickfaden; und
- Stricken des proximalen Stützteils aus demselben Schussfaden und einem zweiten Strickfaden, der den thermoverformbaren Faden aufweist, wobei dieser zweite Strickfaden anstelle des ersten Strickfadens oder zusätzlich zu diesem verwendet wird.

## Claims

1. An orthosis for medical compression/splinting of the lower limb, in the form of a sock, a stocking or a pair of tights,
such orthosis (10) comprising:
- an elastic compressive distal portion (14), adapted to cover the ankle, extending to before the beginning of the calf, at the point where the Achilles tendon joins the calf muscles, said distal portion being made by knitting a knit yarn and a weft yarn, the dimensioning and the nature of the knit and weft yarns as well as the knit structure being selected in such a way to exert in the circumference direction, once the orthosis has been placed on the limb, an elastic return force likely to produce a compression of the limb at a desired therapeutic level of pressure; and
- a splint proximal portion (16), continuing the compressive distal portion and adjacent thereto, so as to envelop, over the periphery thereof, a region of the calf comprised between the level (B1) of the point where the Achilles tendon joins the calf muscles and the level (D) located below the tibial tuberosity;
said splint proximal portion being a deformable tubular portion knitted in continuation with the elastic compressive distal portion,
said orthosis being **characterized in that** the splint proximal portion:
- is essentially non-elastic, and
- is made by knitting a weft yarn and a knit yarn, one of said weft or knit yarns comprising a thermoformable yarn.

2. The orthosis of claim 1, wherein the compressive distal portion is made by knitting a weft yarn and a first knit yarn, and the adjacent proximal portion is made by knitting the same weft yarn and another knit yarn comprising said thermoformable yarn.

3. The orthosis of claim 2, wherein said first knit yarn is a polyamide and/or cotton covered spandex yarn.

4. The orthosis of claim 3, wherein said weft yarn is a polyamide and/or cotton covered spandex yarn.

5. The orthosis of claim 1, wherein the splint proximal portion is a portion having, at the level of the calf maximum circumference, a high rigidity, of 15 ± 2 mmHg/cm (≈ 20 ± 2 mPa/cm).

6. The orthosis of claim 1, wherein the splint proximal portion is a portion having, at the level of the calf maximum circumference, a moderate rigidity, of 5 ± 2 mmHg/cm (≈ 7 ± 2 mPa/cm).

7. The orthosis of claim 1, wherein the elastic compressive distal portion is a low compression portion, adapted to exert a pressure of 10 to 20 mmHg (13 to 27 hPa) at the level of the ankle minimum circumference.

8. The orthosis of claim 1, wherein the elastic compressive distal portion is a moderate compression portion, adapted to exert a pressure of 20 to 30 mmHg (27 to 40 hPa) at the level of the ankle minimum circumference.

9. A method for tailoring an orthosis for medical compression/splinting of the lower limb to the measure of a patient's leg, said method comprising the following steps:
- obtaining an orthosis (10) according to one of claims 1 to 8, in a rough manufactured initial state;
- heating the orthosis to a temperature at least equal to the temperature of activation of the material of the thermoformable yarn for a predetermined duration, the orthosis being then in a freely stretchable state;
- slipping the orthosis on the patient's leg;
- cooling the orthosis in place on the patient's leg ; and
- removing the orthosis in its finished state,
the orthosis in its finished state having a splint proximal portion (16) made rigid by said activation, and whose shape and dimensions are adjusted to the corresponding dimensions of the patient's calf, which allows this splint proximal portion to perfectly fit the shape of the calf.

10. The tailoring method according to claim 9, wherein the step of heating the orthosis precedes the step of slipping on the leg, and wherein the step of heating is implemented by means of a heating form (28) on which the orthosis has been previously placed, the orthosis, once heated, being removed from this form to be slipped on the leg.

11. The tailoring method according to claim 9, wherein the step of slipping on the leg precedes the step of heating the orthosis, and wherein the step of heating is implemented by means of a heating source (30) directed toward the leg on which the orthosis has been previously slipped.

12. A method of manufacturing an orthosis for medical compression / splinting of the lower limb according to one of claims 1 to 8, comprising the following successive steps, performed in continuity with each other:
- knitting the elastic compressive distal portion from a weft yarn and a knit yarn; and
- knitting the splint proximal portion from a weft yarn and a knit yarn, one of said weft or knit yarns of this splint proximal portion comprising a thermoformable yarn.

13. The method of claim 12, comprising the steps of:
- knitting the elastic compressive distal portion from a weft yarn and a first knit yarn; and
- knitting the splint proximal portion from the same weft yarn and a second knit yarn comprising said thermoformable yarn, this second knit yarn being used as a replacement of or in addition to said first knit yarn.
